Europäisches Patentamt

(19) European Patent Office

Office européen des brevets

(11) Publication number: **0 009 893**

**B1**

(12) **EUROPEAN PATENT SPECIFICATION**

(45) Date of publication of patent specification: **30.03.83**

(21) Application number: **79301837.5**

(22) Date of filing: **06.09.79**

(51) Int. Cl.³: **A 61 M 25/02**

(54) **Surgical safety device.**

(30) Priority: **06.09.78 GB 3584478**

(43) Date of publication of application:
**16.04.80 Bulletin 80/8**

(45) Publication of the grant of the patent:
**30.03.83 Bulletin 83/13**

(84) Designated Contracting States:
**AT BE CH DE FR GB IT LU NL SE**

(56) References cited:
**FR - A - 2 371 934**
**GB - A - 1 394 632**
**US - A - 3 288 136**
**US - A - 4 114 626**

(73) Proprietor: **Peters, Joseph Lennox**
**282B Ballard Lane**
**Finchley London N.12 (GB)**

(73) Proprietor: **Wilson, Richard Ylverton**
**Ashlands Church Walk**
**Ulverton Lancashire (GB)**

(73) Proprietor: **Ashford, Colin John**
**9 Pickley Wood Hayes**
**Bromley Kent (GB)**

(72) Inventor: **Peters, Joseph Lennox**
**282B Ballard Lane**
**Finchley London N.12 (GB)**

(74) Representative: **Simpson, Ronald Duncan Innes**
**et al,**
**A.A.Thornton & Co. Northumberland House 303-**
**306 High Holborn**
**London WCIV 7LE (GB)**

Courier Press, Leamington Spa, England

## Surgical safety device

This invention is concerned with surgical equipment, and more particularly a safety device for use with body tubes.

In surgical procedure it is not uncommon for tubes to be inserted into the body of a patient, either through an existing body orifice or an opening specially provided. Such tubes are referred to generally as body tubes and specific examples of such tubes include catheters or cannulae used for blood or other administration purposes, drainage tubing introduced into body cavities, especially after operations, for draining off fluids, and naso-gastric tubes. It is normal practice for body tubes to be secured to the patients skin outside of the body in order to avoid any inadvertent displacement of the tube either into or out of the body. For securing a body tube at the exterior of a patient there has been proposed in GB—A—1394632 a device comprising two relatively rotatable parts with bores which are eccentric with respect to the rotational axis and which may be aligned to allow movement of a body tube through the bores and then moved out of alignment to grip the tube. In another device proposed in FR—A—2371934 a through bore receiving the body tube is defined by a tubular part having an end portion which is deformed inwardly to grip the tube by means comprising an annular screw cap which is threaded onto a collar fixed around the tubular part.

Both these devices have the disadvantage that the body tube is actually gripped over only a very small length, which means that in the case of flexible body tubes, e.g. of silicone rubber, if the device is tightened to hold the tube securely against displacement there is a danger that it will pinch closed the tube, which could have severe traumatic consequences as far as the patient is concerned.

According to the present invention there is provided a surgical safety device to be fixed externally to the body of a patient fitted with a body tube (T), comprising a through bore for receiving the body tube (T), an element for gripping the body tube (T) received in the bore, and means selectively operable to adjust the gripping element either to grip the body tube (T) firmly to prevent unintentional displacement of the body tube into or out of the patient, or to release the body tube (T) to allow the body tube (T) to be pulled freely through the safety device, characterised in that the gripping element is flexible and elongated and extends generally longitudinally of the bore and the adjusting means is operable to twist said element about the bore axis and body tube for gripping the body tube, and untwist the element to release the body tube.

It has been found that the provision of an elongate flexible element which becomes twisted or wound helically around the body tube allows the tube to be gripped firmly and securely without any risk of collapsing the tube since it engages the tube over an extended lengthwise portion and not merely at one position.

In a particular simple and convenient construction the adjusting means comprises a pair of coaxial tubular members which are relatively rotatable, and the gripping element is fast with said members at its opposite ends so as to twist and untwist in response to relative rotation of the tubular members.

Another disadvantage of the previously proposed devices mentioned above is that frictional engagement is relied upon to secure the parts in their adjusted positions, and the tube may become released unintentionally. In a preferred embodiment of the invention releasable locking means is provided to lock the tubular members positively against relative rotation in a direction to untwist the gripping element from an adjusted twisted condition.

These and other preferred features are defined in the appended claims and will become clear from the following description.

One particular form of safety device embodying the invention will now be described by way of example with reference to the accompanying drawing, in which:—

Figure 1 is a side view of the device;

Figure 2 is an axial section through the device;

Figure 3 is a transverse cross-section taken along the line A—A in Figure 2; and

Figure 4 is the same section as Figure 3, but showing the device adjusted to grip a tube.

The safety device illustrated in the drawings is tubular and includes front and rear main parts 1 and 2, respectively, which are cylindrical and coaxial. The front part 1 has an axial bore 3 and a counterbore into which a forwardly protruding boss 4 of the rear part extends. The rear part 2 has an axial bore 5 which aligns with the bore 3 of part 1 and an elongate gripping element 6 of flexible and slightly elastic material is accommodated within the bores 3, 5. The element 6 has tubular end portions 7, 8 which are fixed firmly to the opposed ends of parts 1, 2 by force-fitted sleeves 9, 10 which clamp the end portions against the walls of bores 3, 5. The end portions of the gripping element are interconnected by a plurality, four as shown, of integral strips 11. Thus the gripping element has the form of a tube with four longitudinal slots cut in its wall.

The internal shoulder formed in front part 1 and the forward end of the boss 4 are provided with ratchet teeth 12, 13 which cooperate to permit relative rotation between the front and rear parts 1, 2 in one direction only. At its forward end the front part 1 is provided with holes 14 for thread 15 by means of which the device can be attached to the body of a patient.

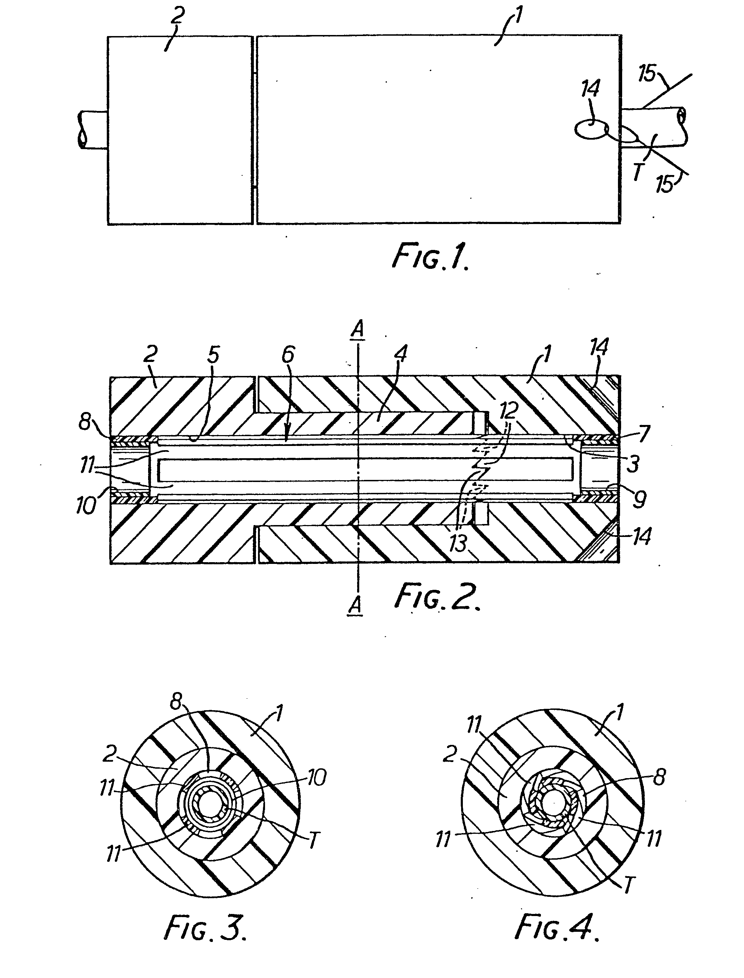

In use of the device, with the device adjusted as seen in Figures 2 and 3, the body tube, for example surgical drainage tube T, to be secured is passed through the device. The forward end of the device is then attached to the patients body by thread 15 passing through holes 14 and stitched to the skin. The device is adjusted to grip firmly the tube T by relatively rotating the front and rear parts 1, 2 in the direction permitted by the ratchet teeth 12, 13. Because the ends of the gripping element 6 are fast with the respective parts 1, 2, the relative rotation between them causes the element to twist so that the strips 11 tend to wind up to extend along generally helical paths and move inwardly to grip the tube T (Figure 4). The strips 11 engage the tube over a substantial length, for example approximately 4 cms, which means that the tube can be held firmly without being crushed or pinched closed. When the device has been tightened the correct amount, the ratchet teeth 12, 13 prevent it loosening.

If, subsequently, it is desired to adjust the position of the tube, such as to shorten the drain, to release the tube the front and rear parts 1, 2 are pulled axially apart to disengage the ratchet teeth 12, 13 and are then turned to loosen the gripping element 6. The tube T can be pulled easily through the device for adjusting its position, after which the device can be tightened again, as described above, to secure the tube once more.

It is to be noted that as the strips 11 are twisted they tend to draw together axially the parts 1, 2 bringing the ratchet teeth into tighter engagement and thereby making the device more secure against inadvertent loosening of the tube. Of course, means other than the ratchet teeth could be provided for locking the two parts 1, 2 in their adjusted positions.

The described safety device can be used with tubes of different diameters and the two parts 1, 2 could be provided with registration marks which would indicate at a glance when the device had been adjusted correctly for each tube diameter. Alternatively the device could be intended for use with one size tube only and be provided with means to limit positively the permitted rotation between parts 1 and 2.

The front and rear parts of the device are conveniently moulded from plastics material and, if required either or both, could be provided with means to assist attachment of the device to a patient, for example a flange plate which could be held in place on the body by a strip of adhesive tape.

Alternatively or additionally a groove could be provided around the periphery of the device for receiving a strap, such as a velcro strap (velcro is a trade mark), to be wrapped around a limb of the patient for attaching the device thereto.

The inner gripping element 6 of the inventive device has been described and illustrated as having its ends fastened to the end portions 7, 8 by force fitted sleeves. However, it will be recognised that other means could be used. For example, the element could be provided with external flanges at its ends which are received in shallow counterbores in the outer ends of the parts 1, 2 and have a splined connection with the parts 1, 2 making its opposite ends fast with the respective parts. The splines could conveniently be formed on the flanges.

Other modifications to the illustrated safety device and alternative forms of device will occur to those readers skilled in the art.

## Claims

1. A surgical safety device to be fixed externally to the body of a patient fitted with a body tube (T), comprising a through bore (3, 5) for receiving the body tube (T), an element (6) for gripping the body tube (T) received in the bore, and means (1, 2) selectively operable to adjust the gripping element (6) either to grip the body tube (T) firmly to prevent unintentional displacement of the body tube into or out of the patient, or to release the body tube (T) to allow the body tube (T) to be pulled freely through the safety device, characterised in that the gripping element (6) is flexible and elongated and extends generally longitudinally of the bore (3, 5), and the adjusting means (1, 2) is operable to twist said element (6) about the bore axis and body tube for gripping the body tube, and untwist the element (6) to release the body tube.

2. A safety device according to claim 1, wherein the said adjusting means comprises a pair of coaxial tubular members (1, 2) which are relatively rotatable, and the gripping element (6) is fast with said members at its opposite ends so as to twist and untwist in response to relative rotation of the tubular members (1, 2).

3. A safety device according to claim 2, wherein releasable locking means (12, 13) is provided to lock said tubular members (1, 2) positively against relative rotation in a direction to untwist the gripping element (6) from an adjusted twisted condition.

4. A safety device according to claim 3, wherein the locking means (12, 13) normally permits relative rotation of the tubular member (1, 2) in one direction only, which direction is in the sense to tighten the grip on the body tube, the locking means (12, 13) being releasable to permits relative rotation of the tubular members (1, 2) to release the body tube (T).

5. A safety device according to claim 4, wherein the locking means comprises a ratchet system including cooperating teeth (12, 13) on the tubular members (1, 2).

6. A safety device according to claim 5, wherein the cooperating ratchet teeth (12, 13) are axially opposed and are disengageable by pulling the tubular members apart axially.

7. A safety device according to claim 6, wherein the ratchet teeth (12, 13) are normally biased into cooperating engagement by the gripping element (6).

8. A safety device as claimed in any one of claims 2 to 7, wherein the tubular members (1, 2) are telescoped together and define the through bore.

9. A safety device as claimed in any preceding claim, wherein the gripping element (6) includes a plurality of parallel strips (11) spaced apart around the periphery of the bore (3, 5) and integrally attached to each other at the ends (7, 8) of the gripping element.

## Patentansprüche

1. Chirurgische Sicherheitseinrichtung für die Befestigung an der Außenseite des Körpers eines mit einem Drain (T) versehenen Patienten, mit einer durchgehenden Bohrung (3, 5) für die Aufnahme des Drains (T), einem Greifelement (6) zum Festhalten des in der Bohrung enthaltenen Drains (T) und Einrichtungen (1, 2), mittels welcher das Greifelement (6) wahlweise einstellbar ist, so daß es den Drain (T) sicher festhält, um eine Verschiebung des Drains in den oder aus dem Körper des Patienten zu verhindern, oder daß es den Drain (T) freigibt, so daß der Drain (T) frei durch die Sicherheitseinrichtung hindurchgezogen werden kann, dadurch gekennzeichnet, daß das Greifelement (6) flexibel und von länglicher Form ist und sich im wesentlichen in Längsrichtung der Bohrung (3, 5) erstreckt, und daß die Einstelleinrichtung (1, 2) zum Zusammendrehen des Greifelements (6) um die Achse der Bohrung und den Drain zum Festhalten desselben und zum Auseinanderdrehen des Greifelements (6) für die Freigabe des Drains betätigbar ist.

2. Sicherheitseinrichtung nach Anspruch 1, dadurch gekennzeichnet, daß die Einstelleinrichtung ein Paar koaxialer rohrförmiger Teile (1, 2) aufweist, welche relativ zueinander verdrehbar sind, und daß das Greifelement (6) an seinen gegenüberliegenden Enden fest mit den beiden Teilen verbunden ist, so daß es durch Verdrehen der rohrförmigen Teile (1, 2) relativ zueinander zusammen- und auseinanderdrehbar ist.

3. Sicherheitseinruchtung nach Anspruch 2, dadurch gekennzeichnet, daß lösbare Verriegelungseinrichtungen (12, 13) vorhanden sind, mittels welcher die rohrförmigen Teile (1, 2) formschlüssig gegen eine Relativdrehung im Sinne des Auseinanderdrehens des Greifelements (6) aus einem zusammengedrehten Zustand verriegelbar sind.

4. Sicherheitseinrichtung nach Anspruch 3, dadurch gekennzeichnet, daß die Verriegelungseinrichtungen (12, 13) im Normalzustand eine Relativdrehung der rohrförmigen Teile in nur einer Richtung im Sinne der Verstärkung des Angriffs am Drain gestatten und zur Freigabe der Relativdrehung der rohrförmigen Teile (1, 2) im Sinne der Freigabe des Drains (T) lösbar sind.

5. Sicherheitseinrichtung nach Anspruch 4, dadurch gekennzeichnet, daß die Verriegelungseinrichtungen eine Ratschenanordnung mit zusammenwirkenden Zähnen (12, 13) an den rohrförmigen Teilen (1, 2) aufweisen.

6. Sicherheitseinrichtung nach Anspruch 5, dadurch gekennzeichnet, daß die zusammenwirkenden Ratschenzähne (12, 13) einander in Axialrichtung gegenüberstehen und durch axiales Auseinanderziehen der rohrförmigen Teile voneinander lösbar sind.

7. Sicherheitseinrichtung nach Anspruch 6, dadurch gekennzeichnet, daß die Ratschenzähne (12, 13) im Normalzustand durch das Greifelement (6) in gegenseitigen Eingriff belastet sind.

8. Sicherheitseinrichtung nach einem der Ansprüche 2 bis 7, dadurch gekennzeichnet, daß die rohrförmigen Teile (1, 2) teleskopartig ineinander geschoben sind und die durchgehende Bohrung umgrenzen.

9. Sicherheitseinrichtung nach einem der vorstehenden Ansprüche, dadurch gekennzeichnet, daß das Greiferlement (6) eine Anzahl von in gegenseitigen Abständen um den Umfang der Bohrung (3, 5) herum angeordneter, paralleler Streifen (11) aufweist, welche an den Enden (7, 8) der Greiferelements einstückig miteinander verbunden sind.

## Revendications

1. Dispositif chirurgical de sécurité devant être fixé extérieurement au corps d'un patient, corps dans lequel est engagé un tube (T) de corps, ce dispositif comprenant un passage (3, 5) recevant le tube (T), un élément (6) de serrage de ce tube (T) le traversant, et un moyen (1, 2) actionnable sélectivement pour ajuster cet élément (6) soit pour serrer énergiquement le tube, de manière à empêcher son déplacement inopiné, d'entrée ou de sortie, par rapport au patient, soit pour relâcher ce tube (T) pour lui permettre d'être tiré librement à travers le dispositif, lequel dispositif est caractérisé en ce que l'élément (6) est flexible et allongé et s'étend sensiblement en longueur du passage, et en ce que le moyen (1, 2) d'ajustage est actionnable pour déformer l'élément (6) en torsion autour de l'axe du passage et du tube (T) afin de le serrer, et de supprimer cette torsion pour le relâcher.

2. Dispositif selon la revendication 1, dans lequel ledit moyen d'ajustage comprend deux pièces tubulaires (1, 2) coaxiales pouvant tourner relativement, et l'élément (6) de serrage leur est fixé par ses extrémités opposées, de manière à être tordu et relâché en réponse à leur rotation relative dans un sens ou dans l'autre.

3. Dispositif selon la revendication 2, dans lequel un moyen (12, 13) de verrouillage libérable permet de bloquer efficacement les pièces tubulaires (1, 2) à l'encontre d'une rotation relative dans le sens de leur relâchement, à partir de leur ajustage en état de torsion.

4. Dispositif selon la revendication 3, dans lequel le moyen de verrouillage (12, 13) permet normalement la rotation des pièces tubulaires (1, 2) dans un seul sens, qui est celui du serrage du tube (T), le moyen de verrouillage étant relâchable pour permettre la rotation relative des pièces (1, 2) de libération du tube (T).

5. Dispositif selon la revendication 4, dans lequel le moyen de verrouillage comprend un dispositif à rochets comprenant des dents coopérantes (12, 13) agencées sur les pièces tubulaires (1, 2).

6. Dispositif selon la revendication 5, dans lequel lesdites dents coopérantes (12, 13) sont opposées en direction axiale et peuvent être mutuellement désengagées séparant les pièces tubulaires (1, 2) en direction axiale.

7. Dispositif selon la revendication 6, dans lequel lesdites dents coopérantes (12, 13) sont normalement rappelées en coopération par l'élément (6) de serrage.

8. Dispositif selon l'une quelconque des revendications 2 à 7, dans lequel les pièces tubulaires (1, 2) peuvent être mutuellement rentrées et constituer ledit passage (3, 5).

9. Dispositif selon l'une quelconque des revendications précédentes, dans lequel l'élément (6) de serrage comprend plusieurs bandes parallèles (11) réparties autour du passage (3, 5) tout en restant solidaires les unes des autres par leurs extrémités (7, 8) sur l'élément de serrage (6).

Fig.1.

Fig.2.

Fig.3.

Fig.4.